# FASCICULE DE BREVET EUROPEEN

(11) **EP 2 417 995 B1**
(45) Date de publication et mention de la délivrance du brevet: **04.03.2015**
(21) Numéro de dépôt: 11174309.2
(22) Date de dépôt: 18.07.2011
(51) Int. Cl.: A61M 16/08, A61M 16/04

(54) **Système d'assistance respiratoire.**
System zur Atmungsunterstützung
Respiratory assistance system

(30) Priorité: 12.08.2010 FR 1003343
(43) Date de publication de la demande: 15.02.2012
(73) Titulaire: Boussignac, Georges, 92160 Antony (FR)
(72) Inventeur: Boussignac, Georges, 92160 Antony (FR)
(74) Mandataire: Coralis Harle

(56) Documents cités:
- DE-A1-102009 010 129
- FR-A1- 2 936 955

## Description

La présente invention a pour objet un système d'assistance respiratoire, notamment destiné à la réanimation d'un patient en état d'arrêt cardiaque.

On connaît déjà des systèmes d'assistance respiratoire qui comportent :
- un dispositif d'assistance respiratoire comprenant :
   ■ un tube, qui forme un canal principal et qui est destiné à être relié, par son extrémité distale, à une voie respiratoire d'un patient pour que le canal principal relie, à l'extérieur, le système respiratoire du patient ;
   ■ des canaux auxiliaires qui débouchent dans le canal principal en amont de l'extrémité distale de celui-ci ; et
   ■ un embout tubulaire monté solidaire, de façon étanche, à l'extrémité proximale du tube et qui peut être relié à un respirateur artificiel, et
- un conduit d'amenée de gaz respiratoire apte à relier l'embout tubulaire à la source de gaz respiratoire pour alimenter en gaz respiratoire les canaux auxiliaires.

FR 2 936 955 A1 divulgue un tel système.

Ainsi, en cas d'arrêt cardiaque chez un patient, il est possible de ventiler les poumons de ce dernier avec du gaz respiratoire en utilisant un tel système d'assistance respiratoire. Ce système peut être mis en oeuvre de la façon connue suivante :
- dans un premier temps, on introduit une partie du tube dans la trachée du patient. Le dispositif d'assistance respiratoire est ensuite relié, au niveau de son embout tubulaire, à une source de gaz respiratoire (par exemple une bouteille d'oxygène), par l'intermédiaire du conduit d'amenée. Le patient est alors ventilé en oxygène provenant de ladite source, après avoir transité par le conduit d'amenée et par les canaux longitudinaux. Au cours de l'insufflation continue d'oxygène, du personnel soignant exerce des compressions et des décompressions alternées sur la cage thoracique du patient ce qui permet une ventilation de ce dernier (encore désignée oxygénation passive) ; et
- après la remise en marche du coeur, le conduit d'amenée est déconnecté de l'embout tubulaire (et donc du dispositif d'assistance respiratoire). Un respirateur artificiel peut alors être raccordé directement à l'embout tubulaire du dispositif d'assistance respiratoire.

Cependant, dans l'urgence de la situation, des erreurs peuvent être commises par le personnel soignant. En particulier, il arrive que l'embout tubulaire, du tube introduit dans la trachée du patient, soit accidentellement relié à un respirateur artificiel avant même que le conduit d'amenée ne soit déconnecté dudit embout.

Dans ce dernier cas, les poumons du patient sont ventilés par de l'oxygène provenant à la fois de la source extérieure et du respirateur artificiel. En raison du fait que le respirateur artificiel est un système fermé, cela conduit à une augmentation de pression à l'intérieur desdits poumons à même de compromettre la santé, déjà fragile, du patient.

La présente invention a pour objet de remédier à cet inconvénient et, notamment, d'éviter l'apparition d'une surpression dans le système respiratoire d'un patient soumis à une insufflation continue de gaz respiratoire.

A cette fin, selon l'invention, le système d'assistance respiratoire qui comporte :
- un dispositif d'assistance respiratoire comprenant :
   ■ un corps tubulaire, qui forme un canal principal et qui est destiné à être relié, par son extrémité distale, à une voie respiratoire d'un patient pour que le canal principal relie, à l'extérieur, le système respiratoire du patient ;
   ■ au moins un canal auxiliaire qui débouche dans le canal principal en amont de l'extrémité distale de celui-ci ; et
   ■ un embout tubulaire monté solidaire, de façon étanche, à l'extrémité proximale du corps tubulaire, et
- un conduit d'amenée de gaz respiratoire apte à relier ledit embout tubulaire à une source de gaz respiratoire, de manière à alimenter en gaz respiratoire ledit canal auxiliaire,
   est remarquable en ce que ledit système comporte des moyens, formant obstacle, qui s'opposent au montage d'un élément de raccord d'un dispositif médical externe audit embout tubulaire, lorsque ce dernier est relié à ladite source de gaz respiratoire par l'intermédiaire dudit conduit d'amenée.

Ainsi, grâce à l'invention, aucun dispositif médical externe (par exemple un respirateur artificiel) ne peut être raccordé à l'embout tubulaire, dans le cas où celui-ci est déjà relié à une source de gaz respiratoire. Dans le cas d'un arrêt cardiaque chez un patient, la ventilation du patient, obtenue par l'insufflation continue de gaz respiratoire combinée aux compressions et décompressions effectuées par le personnel soignant qui engendrent une pression intra thoracique positive (pendant les compressions) et négative (pendant les décompressions) permettant d'obtenir une meilleure hémodynamique, est ainsi entièrement sécurisée. Il n'existe en effet aucun risque d'apparition d'une suppression à l'intérieur des voies respiratoires du patient, surpression provoquée par exemple par un apport supplémentaire en gaz respiratoire provenant d'un dispositif médical externe de type système fermé (par exemple un respirateur artificiel).

En outre, la présente invention permet de simplifier la prise en charge d'un patient en état d'arrêt cardiaque, de sorte qu'une seule personne peut s'occuper de celui-ci.

En outre, lesdits moyens formant obstacle sont avantageusement montés à l'extrémité distale dudit conduit d'amenée.

Ainsi, après la déconnexion du conduit d'amenée de l'embout tubulaire (par exemple à la remise en marche du coeur de celui-ci) qui provoque l'arrêt de l'insufflation continue de gaz respiratoire, l'embout tubulaire du dispositif d'assistance respiratoire est libéré des moyens formant obstacle et peut, dès lors, être raccordé à un dispositif médical externe. Dans ce cas, les moyens formant obstacle sont amovibles par rapport à l'embout tubulaire du dispositif d'assistance respiratoire. Ils ne coopèrent avec l'embout tubulaire que lors de son raccord au conduit d'amenée.

Il va de soi que, en variante, les moyens formant obstacle pourraient être montés directement sur le dispositif d'assistance respiratoire, ou bien encore être indépendants et amovibles de ce dernier et du conduit d'amenée.

Dans une forme de réalisation conforme à la présente invention, lesdits moyens formant obstacle comportent une gouttière ouverte qui définit un logement apte à recevoir au moins une partie dudit embout tubulaire, lorsque ce dernier est relié à ladite source de gaz respiratoire par ledit conduit d'amenée. En variante, on pourrait bien entendu envisager que lesdits moyens formant obstacle comportent une gouttière fermée.

Ainsi, lorsqu'au moins une partie de l'embout tubulaire est logée dans la gouttière, cette dernière forme une surépaisseur sur la partie recouverte de la paroi latérale externe dudit embout tubulaire, ce qui empêche tout raccordement à un dispositif médical externe, au cours de l'insufflation de gaz respiratoire provenant de ladite source.

En outre, ladite gouttière peut être reliée audit conduit d'amenée, au niveau de son extrémité distale, par des moyens de liaison. Ces derniers peuvent comporter une bague, montée libre en rotation autour du conduit d'amenée, et au moins un bras de liaison, solidaire de ladite bague et de ladite gouttière.

Par ailleurs, lorsque ledit embout tubulaire est relié à ladite source de gaz respiratoire par l'intermédiaire dudit conduit d'amenée, ladite gouttière se prolonge longitudinalement, de préférence, au moins jusqu'à l'extrémité proximale dudit embout tubulaire, de manière à s'opposer au montage d'un élément de raccord d'un dispositif médical externe.

De préférence, ladite gouttière présente une forme semi-cylindrique, pour assurer un maintien convenable dudit embout, lorsque le conduit d'amenée est raccordé à celui-ci. Dans ce dernier cas, l'embout tubulaire et la gouttière, dans laquelle il est logé, sont sensiblement concentriques.

Selon une autre forme de réalisation conforme à la présente invention :
- ledit embout tubulaire comporte :
   ■ un élément tubulaire latéral saillant destiné à être raccordé audit conduit d'amenée de gaz respiratoire ; et
   ■ une chambre annulaire étanche interne dans laquelle débouchent ledit élément latéral saillant et ledit canal auxiliaire ; et
- la gouttière comporte une ouverture destinée à être traversée par ledit élément latéral saillant, lorsque ledit embout tubulaire est relié à ladite source par le conduit d'amenée.

En outre, l'extrémité distale du conduit d'amenée peut comporter un écrou de fixation, monté libre en rotation autour dudit conduit, qui est apte à coopérer avec des moyens complémentaires de fixation agencés à l'extrémité libre dudit élément latéral saillant.

Les figures du dessin annexé feront bien comprendre comment l'invention peut être réalisée. Sur ces figures, des références identiques désignent des éléments semblables.
La figure 1 est une vue générale d'un exemple de système d'assistance respiratoire, conforme à la présente invention, formé d'un dispositif d'assistance respiratoire et d'un conduit d'amenée de gaz respiratoire. Sur cette figure, le conduit d'amenée est représenté raccordé au dispositif d'assistance respiratoire.
La figure 2 illustre partiellement, dans une coupe schématique longitudinale, le dispositif d'assistance respiratoire de la figure 1, lorsqu'il n'est pas raccordé au conduit d'amenée.
La figure 3 est une coupe schématique transversale, selon la ligne III-III de la figure 2, du dispositif d'assistance respiratoire de la figure 1.
La figure 4 représente, dans une vue schématique partielle, l'embout tubulaire et les moyens formant obstacle du système de la figure 1, lorsque le conduit d'amenée est déconnecté dudit embout tubulaire.
La figure 5 montre, dans une vue schématique en bout, l'embout tubulaire et les moyens formant obstacle du système de la figure 1, lorsque le conduit d'amenée est relié audit embout tubulaire.

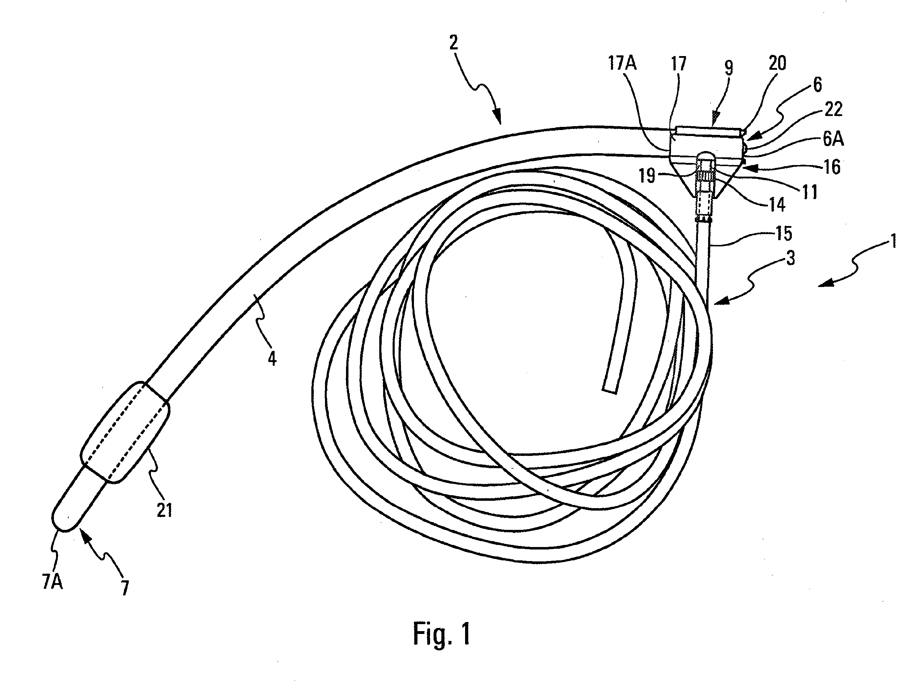

Sur la figure 1, on a représenté, de façon schématique, un exemple de système d'assistance respiratoire 1, conforme à la présente invention, qui comporte un dispositif d'assistance respiratoire 2 et un conduit 3 d'amenée de gaz respiratoire destiné à être raccordé à une source de gaz respiratoire (non représentée), par exemple une bouteille d'oxygène.

Dans cet exemple, le dispositif 2 se présente sous la forme d'une sonde endotrachéale avec ballonnet, notamment utilisée en cas d'arrêt cardiaque chez un patient. Lorsque cette sonde est raccordée par le conduit d'amenée 3 à une source de gaz respiratoire, elle forme un dispositif d'oxygénation passive.

Bien entendu, la présente invention n'est en rien limitée à cet exemple, de sorte que le dispositif d'assistance respiratoire pourrait également prendre la forme d'un dispositif de ventilation spontanée en pression positive CPAP utilisé en dispositif d'oxygénation passive sur un patient en état d'arrêt cardiaque, d'une sonde endotrachéale pédiatrique, d'un dispositif supraglottique (par exemple vendu commercialement sous le nom de KING SYSTEMS, ou bien encore de COMBITUBE) , d'une sonde de monitorage des gaz, d'une sonde endobronchique, d'une sonde d'intubation anatomique pour enfant, etc ...

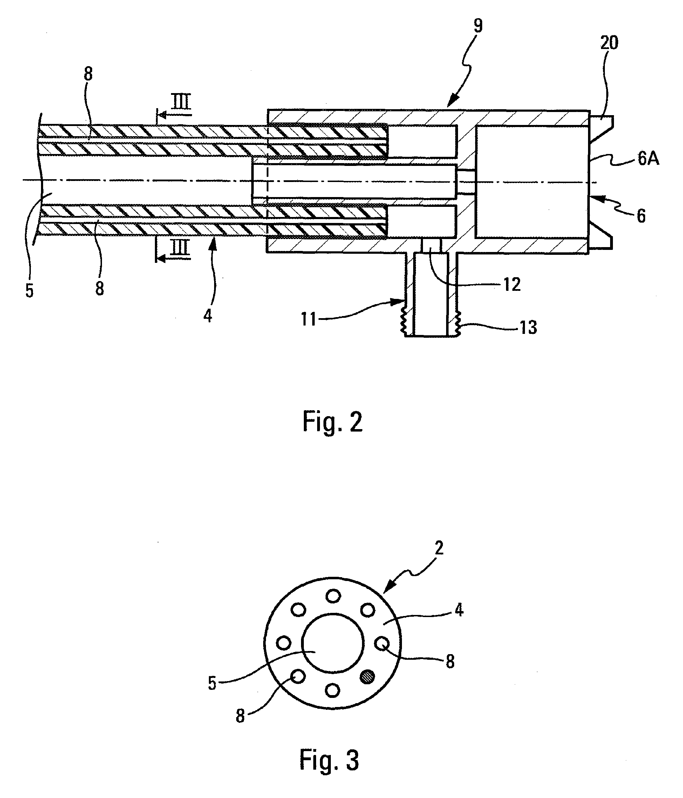

Comme le montrent les figures 1 et 2, le dispositif 2 comporte, de façon connue, un tube 4, souple ou préformé (pour s'adapter à la morphologie du patient pris en charge), délimitant un canal principal 5 ayant un orifice proximal 6A et un orifice distal 7A, respectivement aux extrémités proximale 6 et distale 7 dudit tube 4.

Ainsi, le canal principal 5 est capable d'assurer le passage entre les orifices proximal 6A et distal 7A, dont l'un (orifice distal 7A) est destiné à se trouver à l'intérieur des voies respiratoires du patient et l'autre (orifice proximal 6A) est destiné à se trouver à l'extérieur de celles-ci. Cet orifice proximal 6A peut déboucher à l'air libre et, dans ce cas, le patient peut inspirer de l'air frais et expirer de l'air vicié à travers le canal principal 5.

En outre, dans l'épaisseur de la paroi du tube 4, sont ménagés des canaux auxiliaires longitudinaux 8, s'étendant sur la presque totalité de la longueur du tube 4 et destinés à être reliés, pour au moins certains d'entre eux, à une source de gaz respiratoire sous pression. Les canaux longitudinaux 8 débouchent dans le canal principal 5, au voisinage de son extrémité distale 7.

Comme le montre la figure 3, les canaux auxiliaires 8 sont disposés régulièrement autour de l'axe du tube 4. Au moins un des canaux auxiliaires 8 (représenté en grisé sur la figure 3) peut être spécialisé pour apporter un fluide médical (par exemple un médicament).

Le tube 4 du dispositif 2 peut être réalisé en toute matière déjà utilisée dans les sondes respiratoires, par exemple en un chlorure de polyvinyle, avec un éventuel revêtement en silicone ou en acier permettant les injections à pression élevée.

En outre, un ballonnet gonflable 21 est monté sur la paroi latérale externe du tube 4, dans un voisinage de l'extrémité distale 7 de ce dernier. Lorsque le tube 4 est positionné dans la trachée du patient, le ballonnet gonflable 21 permet, une fois gonflé, d'assurer l'étanchéité entre la trachée et le contour extérieur du tube 4.

Le dispositif 2 comporte également un embout tubulaire 9 monté solidaire, de façon étanche, à l'extrémité proximale 6 du tube 4. L'embout 9 est, par exemple, apte à raccorder le tube 4 à un respirateur artificiel (non représenté) au niveau de son extrémité proximale 6.

L'embout 9 comporte une chambre annulaire interne 10, formée dans la zone de raccordement du tube 4 et de l'embout 9 et destinée à être reliée à la source de gaz respiratoire, par l'intermédiaire du conduit d'amenée 3.

Une fois relié à la source de gaz respiratoire, la chambre annulaire 10 devient étanche à l'air extérieur ambiant. Dans ce dernier cas, la chambre annulaire 10 n'est traversée que par du gaz respiratoire provenant de ladite source et les extrémités proximales des canaux auxiliaires 8, qui débouchent dans la chambre annulaire 10, sont alors alimentées par du gaz respiratoire.

Comme le montrent les figures 1 et 2, l'embout tubulaire 9 comprend également un élément tubulaire latéral saillant 11 qui communique avec la chambre annulaire 10 par l'intermédiaire d'un orifice 12 ménagé dans la paroi latérale de l'embout 9.

En outre, l'élément tubulaire saillant 11 est adapté pour être raccordé au conduit d'amenée 3. Pour cela, il comporte un filetage 13 sur sa paroi latérale externe, qui est destiné à coopérer avec un écrou complémentaire 14 monté libre en rotation sur l'extrémité distale 15 du conduit d'amenée 3.

Selon l'invention, tel qu'illustré sur les figures, le système 1 comporte des moyens 16, formant obstacle, qui s'opposent au montage d'un élément de raccord d'un dispositif médical externe, par exemple un respirateur artificiel, à l'embout tubulaire 9 du dispositif 2, lorsque ce dernier est raccordé à la source de gaz respiratoire par l'intermédiaire du conduit d'amenée 3.

Dans l'exemple décrit, les moyens 16 formant obstacle sont montés à l'extrémité distale 15 du conduit d'amenée 3. Bien entendu, en variante, ces moyens pourraient être disposés au voisinage de l'extrémité proximale du dispositif d'assistance respiratoire.

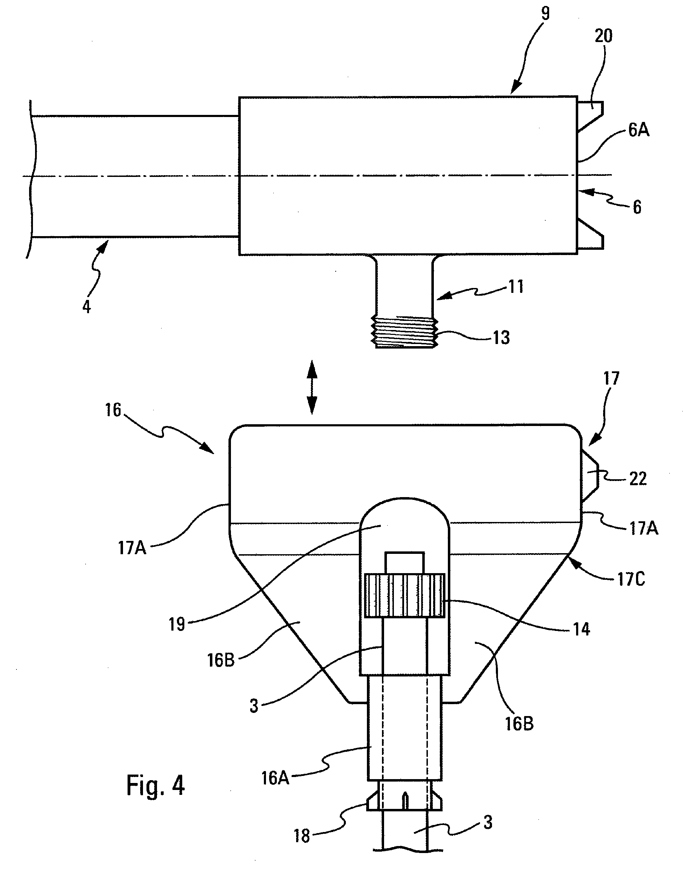

Comme le montrent les figures 1, 4 et 5, les moyens 16 formant obstacle comportent :
- une gouttière semi-cylindrique 17, dont les deux extrémités longitudinales 17A sont ouvertes. L'intérieur de la gouttière 17 définit un logement de forme complémentaire à celle de l'embout tubulaire 9 ;
- une bague 16A, montée libre en rotation autour du conduit d'amenée 3 ; et
- deux bras de liaison 16B qui relient, de façon rigide, le fond 17C de la gouttière 17 à la bague 16A.

Ainsi, les moyens 16 formant obstacle sont montés libres en rotation autour du conduit 3, lorsque ce dernier n'est pas raccordé à l'embout 9. En outre, dans ce dernier cas, ils peuvent également se déplacer suivant la direction longitudinale du conduit 3. Toutefois, un tel déplacement longitudinal est limité, du côté distal, par des butées 18 sous forme d'ailette rapportées sur le conduit d'amenée 3 et, du côté proximal, par l'écrou 14.

Par ailleurs, dans le fond 17C de la gouttière 17, est pratiquée une ouverture 19 permettant le passage, au travers celle-ci, de l'élément latéral saillant 11 lors du raccordement de ce dernier au conduit d'amenée 3.

En outre, les dimensions de la section transversale extérieure de l'embout 9 sont avantageusement inférieures à celles de la section transversale intérieure de la gouttière 17, pour que l'embout tubulaire 9 puisse se loger aisément à l'intérieur de cette dernière.

Ainsi, lorsque l'élément latéral 11 est fixé au conduit 3 au moyen de l'écrou 14, après avoir traversé l'ouverture 19, l'embout 9 se retrouve logé à l'intérieur de la gouttière 17.

Dans un tel agencement, la paroi latérale 17B de la gouttière 17 recouvre :
- dans le sens de la longueur (définie selon la direction longitudinale de l'embout 9), l'intégralité de l'embout 9. Avantageusement, la paroi latéral s'étend longitudinalement au-delà de l'extrémité distale 6 de l'embout 9 ; et
- dans le sens de la hauteur (définie selon une direction orthogonale à la direction longitudinale), une partie de l'embout 9.

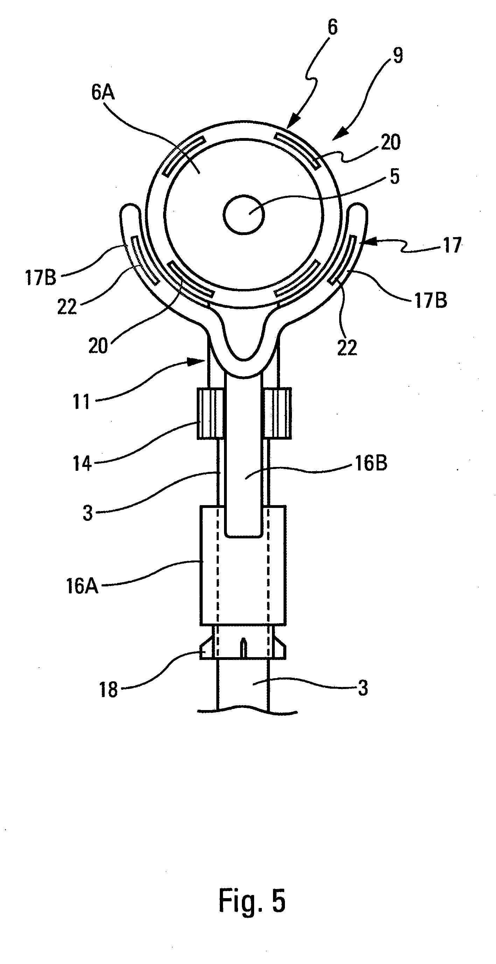

Comme le montre la figure 5, la gouttière 17, une fois rapportée sur l'embout tubulaire 9, entraîne une augmentation, artificielle et réversible, du diamètre de la section transversale extérieure de l'embout 9.

Ainsi, le raccordement d'un élément de raccord d'un dispositif médical, tel qu'un respirateur artificiel, est irréalisable, la section transversale de l'élément de raccord du dispositif externe étant destinée à coopérer, avec ajustement, avec celle de l'embout tubulaire 9, dépourvu de gouttière.

Par ailleurs, l'embout tubulaire 9 comporte également des ailettes 20, agencées sur le pourtour de l'orifice proximale 6A, dans le prolongement de la paroi latérale de l'embout 9, pour éviter toute obstruction de celui-là.

De même, les moyens formant obstacle 16 comportent des ailettes 22, par exemple disposées à l'extrémité longitudinale proximale de la gouttière 17, de manière à prévenir l'obstruction de l'orifice proximale 6A lorsque ledit embout 9 est relié à ladite source de gaz respiratoire par l'intermédiaire du conduit 3.

Il va de soi que de telles ailettes pourraient être disposées soit uniquement sur les moyens formant obstacle, soit, au contraire, uniquement sur l'embout tubulaire.

En cas d'arrêt cardiaque chez un patient, le personnel soignant introduit, dans un premier temps, une partie du tube 4 dans la trachée du patient.

Le conduit d'amenée 3 est ensuite raccordé à l'élément tubulaire latéral saillant 11, de manière à relier l'embout tubulaire 9 à la source de gaz respiratoire et ainsi alimenter en gaz respiratoire les canaux auxiliaires 8 appropriés. Dans cet agencement, la gouttière 17 des moyens 16 formant obstacle entoure partiellement l'embout tubulaire 9, de sorte qu'aucun respirateur artificiel ne peut être connecté à ce dernier lors de l'insufflation continue de gaz respiratoire.

La ventilation du patient, obtenue par l'insufflation continue de gaz respiratoire du système 1 combinée aux compressions et décompressions effectuées par le personnel soignant qui engendrent une pression intra thoracique positive (pendant les compressions) et négative (pendant les décompressions) permettant d'obtenir une meilleure hémodynamique, est ainsi entièrement sécurisée.

Il n'existe aucun risque d'apparition d'une suppression à l'intérieur des voies respiratoires dudit patient lors de l'insufflation continue de gaz respiratoire.

Après la remise en marche du coeur, le conduit d'amenée 3 est déconnecté de l'élément tubulaire latéral 11, afin de permettre le branchement d'un respirateur artificiel sur l'embout tubulaire 9 du dispositif 2.

L'invention a été décrite en relation avec un exemple de moyens formant obstacle qui se présentent notamment sous la forme d'une gouttière semi-cylindrique et qui sont montés à l'extrémité distale du conduit d'amenée. Il va de soi que l'invention n'est pas limitée à cette forme de réalisation, mais comprend également toute autre forme appropriée des moyens formant obstacle destinés à empêcher le raccord d'un dispositif médical externe à l'embout tubulaire, lorsque ce dernier est relié à une source de gaz respiratoire par le conduit d'amenée. En outre, bien qu'elle soit particulièrement adaptée au cas des arrêts cardiaques, la présente invention n'est nullement restreinte à cette dernière application.

## Revendications

1. Système d'assistance respiratoire qui comporte :
- un dispositif d'assistance respiratoire (2) comprenant :
▪ un corps tubulaire (4), qui forme un canal principal (5) et qui est destiné à être relié, par son extrémité distale (7), à une voie respiratoire d'un patient pour que le canal principal (5) relie, à l'extérieur, le système respiratoire du patient ;
▪ au moins un canal auxiliaire (8) qui débouche dans le canal principal (5) en amont de l'extrémité distale (7) de celui-ci ; et
▪ un embout tubulaire (9) monté solidaire, de façon étanche, à l'extrémité proximale (6) du corps tubulaire (4), et
- un conduit (3) d'amenée de gaz respiratoire apte à relier ledit embout tubulaire (9) à une source de gaz respiratoire, de manière à alimenter en gaz respiratoire ledit canal auxiliaire (8),
**caractérisé en ce que** ledit système (1) comporte des moyens (16), formant obstacle, qui s'opposent au montage d'un élément de raccord d'un dispositif médical externe audit embout tubulaire (9), lorsque ce dernier est relié à ladite source de gaz respiratoire par l'intermédiaire dudit conduit d'amenée (3).

2. Système d'assistance respiratoire selon la revendication 1, **caractérisé en ce que** lesdits moyens (16) formant obstacle sont montés à l'extrémité distale (15) dudit conduit d'amenée (3).

3. Système d'assistance respiratoire selon la revendication 2, **caractérisé en ce que** lesdits moyens (16) formant obstacle comportent une gouttière ouverte (17) qui définit un logement apte à recevoir au moins une partie dudit embout tubulaire (9), lorsque ce dernier est relié à ladite source de gaz respiratoire par ledit conduit d'amenée (3).

4. Système d'assistance respiratoire selon la revendication 3, **caractérisé en ce que** ladite gouttière (16) est reliée audit conduit d'amenée (3), au niveau de son extrémité distale (15), par des moyens de liaison (16A, 16B).

5. Système d'assistance respiratoire selon la revendication 4, **caractérisé en ce que** lesdits moyens de liaison (16A, 16B) comportent une bague (16A), montée libre en rotation autour du conduit d'amenée (3), et au moins un bras de liaison (16B), solidaire de ladite bague (16A) et de ladite gouttière (17).

6. Système d'assistance respiratoire selon l'une des revendications 3 à 5,
**caractérisé en ce que**, lorsque ledit embout tubulaire (9) est relié à ladite source de gaz respiratoire par l'intermédiaire dudit conduit d'amenée (3), ladite gouttière (17) se prolonge longitudinalement au moins jusqu'à l'extrémité proximale (6) dudit embout tubulaire (9), de manière à s'opposer au montage d'un élément de raccord d'un dispositif médical externe.

7. Système d'assistance respiratoire selon l'une des revendications 3 à 6,
**caractérisé en ce que** ladite gouttière (17) présente une forme semi-cylindrique.

8. Système d'assistance respiratoire selon l'une des revendications 3 à 7,
**caractérisé :**
- **en ce que** ledit embout tubulaire (9) comporte :
▪ un élément tubulaire latéral saillant (11) destiné à être raccordé audit conduit (3) d'amenée de gaz respiratoire ; et
▪ une chambre annulaire étanche interne (10) dans laquelle débouchent ledit élément latéral saillant (11) et ledit canal auxiliaire (8) ; et
- **en ce que** la gouttière (17) comporte une ouverture (19) destinée à être traversée par ledit élément tubulaire latéral saillant (11), lorsque ledit embout tubulaire (9) est relié à ladite source par le conduit d'amenée (3).

9. Système d'assistance respiratoire selon la revendication 8,
**caractérisé en ce que** l'extrémité distale (15) du conduit d'amenée (3) comporte un écrou de fixation (14), monté libre en rotation autour dudit conduit (3), qui est apte à coopérer avec des moyens complémentaires de fixation (13) agencés à l'extrémité libre dudit élément latéral saillant (11).

10. Système d'assistance respiratoire selon l'une des revendications 1 à 9,
**caractérisé en ce que** lesdits moyens formant obstacle (16) comportent au moins une ailette (22) pour prévenir toute obstruction de l'extrémité proximale dudit embout tubulaire (9), lorsque celui-ci est relié à ladite source de gaz respiratoire par l'intermédiaire dudit conduit d'amenée (3).

11. Système d'assistance respiratoire selon l'une des revendications 1 à 10,
**caractérisé en ce que** ledit embout (9) comportent, à son extrémité proximale, au moins une ailette (20), pour prévenir toute obstruction de celle-ci.

## Patentansprüche

1. System zur Atmungsunterstützung, welches aufweist:
- eine Atmungsunterstützungsvorrichtung (2), welche umfasst:
• einen rohrförmigen Körper (4), welcher einen Hauptkanal (5) bildet und welcher dazu bestimmt ist, über sein distales Ende (7) mit einem Atemweg eines Patienten verbunden zu werden, damit der Hauptkanal (5) das Atemsystem des Patienten mit der Außenumgebung verbindet;
• mindestens einen Hilfskanal (8), welcher in den Hauptkanal (5) stromaufwärts des distalen Endes (7) desselben mündet; und
• ein rohrförmiges Ansatzstück (9), das einstückig und auf dichte Weise an dem proximalen Ende (6) des rohrförmigen Körpers (4) angebracht ist, und
- einen Atemgas-Zufuhrkanal (3), welcher geeignet ist, das rohrförmige Ansatzstück (9) mit einer Atemgasquelle zu verbinden, um den Hilfskanal (8) mit Atemgas zu speisen;
**dadurch gekennzeichnet, dass** das System (1) Mittel (16), die ein Hindernis bilden, aufweist, welche der Anbringung eines Anschlusselements einer externen medizinischen Vorrichtung an dem rohrförmigen Ansatzstück (9) im Wege stehen, wenn dieses Letztere über den Zufuhrkanal (3) mit der Atemgasquelle verbunden ist.

2. System zur Atmungsunterstützung nach Anspruch 1, **dadurch gekennzeichnet, dass** die ein Hindernis bildenden Mittel (16) am distalen Ende (15) des Zufuhrkanals (3) angebracht sind.

3. System zur Atmungsunterstützung nach Anspruch 2, **dadurch gekennzeichnet, dass** die ein Hindernis bildenden Mittel (16) eine offene Rinne (17) aufweisen, welche eine Aufnahme definiert, die geeignet ist, wenigstens einen Teil des rohrförmigen Ansatzstücks (9) aufzunehmen, wenn dieses Letztere über den Zufuhrkanal (3) mit der Atemgasquelle verbunden ist.

4. System zur Atmungsunterstützung nach Anspruch 3, **dadurch gekennzeichnet, dass** die Rinne (16) mit dem Zufuhrkanal (3) an dessen distalem Ende (15) durch Verbindungsmittel (16A, 16B) verbunden ist.

5. System zur Atmungsunterstützung nach Anspruch 4, **dadurch gekennzeichnet, dass** die Verbindungsmittel (16A, 16B) eine Hülse (16A), die frei drehbar um den Zufuhrkanal (3) angebracht ist, und mindestens einen Verbindungsarm (16B), der mit der Hülse (16A) und der Rinne (17) fest verbunden ist, aufweisen.

6. System zur Atmungsunterstützung nach einem der Ansprüche 3 bis 5, **dadurch gekennzeichnet, dass**, wenn das rohrförmige Ansatzstück (9) über den Zufuhrkanal (3) mit der Atemgasquelle verbunden ist, die Rinne (17) sich in Längsrichtung mindestens bis zum proximalen Ende (6) des rohrförmigen Ansatzstücks (9) erstreckt, so dass sie der Anbringung eines Anschlusselements einer externen medizinischen Vorrichtung im Wege steht.

7. System zur Atmungsunterstützung nach einem der Ansprüche 3 bis 6, **dadurch gekennzeichnet, dass** die Rinne (17) eine Halbzylinderform aufweist.

8. System zur Atmungsunterstützung nach einem der Ansprüche 3 bis 7, **gekennzeichnet:**
- **dadurch, dass** das rohrförmige Ansatzstück (9) aufweist:
• ein vorstehendes seitliches rohrförmiges Element (11), das dazu bestimmt ist, an den Atemgas-Zufuhrkanal (3) angeschlossen zu werden; und
• eine innere dichte ringförmige Kammer (10), in welche das vorstehende seitliche Element (11) und der Hilfskanal (8) münden; und
- dadurch, dass die Rinne (17) eine Öffnung (19) aufweist, die dazu bestimmt ist, von dem vorstehenden seitlichen rohrförmigen Element (11) durchquert zu werden, wenn das rohrförmige Ansatzstück (9) über den Zufuhrkanal (3) mit der Quelle verbunden ist.

9. System zur Atmungsunterstützung nach Anspruch 8, **dadurch gekennzeichnet, dass** das distale Ende (15) des Zufuhrkanals (3) eine Befestigungsmutter (14) aufweist, die frei drehbar um den Kanal (3) angebracht ist und die geeignet ist, mit komplementären Befestigungsmitteln (13) zusammenzuwirken, die an dem freien Ende des vorstehenden seitlichen Elements (11) angeordnet sind.

10. System zur Atmungsunterstützung nach einem der Ansprüche 1 bis 9, **dadurch gekennzeichnet, dass** die ein Hindernis bildenden Mittel (16) mindestens einen Flügel (22) aufweisen, um jede Versperrung des proximalen Endes des rohrförmigen Ansatzstücks (9) zu verhindern, wenn dieses über den Zufuhrkanal (3) mit der Atemgasquelle verbunden ist.

11. System zur Atmungsunterstützung nach einem der Ansprüche 1 bis 10, **dadurch gekennzeichnet, dass** das Ansatzstück (9) an seinem proximalen Ende mindestens einen Flügel (20) aufweist, um jede Versperrung desselben zu verhindern.

## Claims

1. A respiratory assistance system comprising:
a respiratory assistance device (2) comprising:
a tubular body (4), forming a main channel (5), intended for being connected, through its distal end (7), to an airway of a patient so that the main channel (5) connects the patient's breathing system to the outside;
at least one auxiliary channel (8) that opens into the main channel (5) upstream of the distal end (7) of the latter; and
a tubular nozzle (9) integrally mounted, tightly, at the proximal end (6) of the tubular body; and
a respiratory gas supply duct (3) able to connect said tubular nozzle (9) to a source of respiratory gas, so as to supply respiratory gas to said auxiliary channel (8),
**characterized in that** said system (1) comprises obstacle forming means (16), preventing assembly of a connecting member of an external medical device to said tubular nozzle (9), when the latter is connected to said source of respiratory gas by means of said supply duct (3).

2. A respiratory assistance system according to claim 1, **characterized in that** said obstacle forming means (16) are mounted at the distal end (15) of said supply duct (3).

3. A respiratory assistance system according to claim 2, **characterized in that** said obstacle forming means (16) comprise an opened groove (17) defining a housing able to receive at least one part of said tubular nozzle (9), when the latter is connected to said source of respiratory gas by said supply duct (3).

4. A respiratory assistance system according to claim 3, **characterized in that** said groove (16) is connected to said supply duct (3), at the level of its distal end (15), by linking means (16A, 16B).

5. A respiratory assistance system according to claim 4, **characterized in that** said linking means (16A, 16B) comprise a ring (16A), being free rotatably mounted around the supply duct (3), and at least one linking arm (16B), integral with said ring (16A) and with said groove (17).

6. A respiratory assistance system according to one of claims 3 to 5, **characterized in that** when said tubular nozzle (9) is connected to said source of respiratory gas by means of said supply duct (3), said groove (17) longitudinally extends at least up to the proximal end (6) of said tubular nozzle (9), so as to prevent assembly of a connecting member of an external medical device.

7. A respiratory assistance system according to one of claims 3 to 6, **characterized in that** said groove (17) has a semi-cylindrical shape.

8. A respiratory assistance system according to one of claims 3 to 7, **characterized in that** said tubular nozzle (9) comprises:
a projecting side tubular member (11) intended for being connected to said respiratory gas supply duct (3); and
an internal tight annular chamber (10) where said projecting side member (11) and said auxiliary channel (8) open into; and
**in that** the groove (17) comprises an opening (19) intended for being crossed through by said projecting side tubular member (11), when said tubular nozzle (9) is connected to said source by means of the supply duct (3).

9. A respiratory assistance system according to claim 8, **characterized in that** the distal end (15) of the supply duct (3) comprises a fastening nut (14), being free rotatably mounted about said duct (3), being able to cooperate with complementary fastening means (13) arranged at the free end of said projecting side member (11).

10. A respiratory assistance system according to one of claims 1 to 9, **characterized in that** said obstacle forming means (16) comprise at least one fin (22) for preventing any obstruction of the proximal end of said tubular nozzle (9), when the latter is connected to said source of respiratory gas by means of said supply duct (3).

11. A respiratory assistance system according to one of claims 1 to 10, **characterized in that** said nozzle (9) comprises, at its proximal end, at least one fin (20), for preventing any obstruction of the latter.
